# EUROPEAN PATENT APPLICATION

(11) **EP 4 817 584 A1**
(43) Date of publication of application: **07.10.2026**
(21) Application number: 24898277.9
(22) Date of filing: 19.09.2024
(51) Int. Cl.: A61H 9/00, A61N 1/40, A61N 1/32, A61N 1/06, A61N 1/08, A61M 1/08

(54) **SUCTION HANDPIECE AND SKIN CARE DEVICE INCLUDING SAME**

(30) Priority: 28.11.2023 KR 20230168288
(71) Applicant: CLASSYS INC., Seoul 06220 (KR)
(72) Inventor: YIM, Dae Uk, Hwaseong-si Gyeonggi-do 18394 (KR); KO, Min Soo, Seoul 01632 (KR); HAN, Jin Teak, Seoul 05502 (KR); PARK, Sung Ho, Gunpo-si Gyeonggi-do 15876 (KR)
(74) Representative: Witte, Weller & Partner Patentanwälte mbB
(86) International application number: PCT/KR2024/096175
(87) International publication number: WO 2025/116685

(57) **Abstract**

The present invention relates to a suction handpiece and a skin care device including same, the handpiece comprising: a handpiece body part having a negative pressure formation space open at a surface thereof that is to come into contact with the skin; an air suction pipe member positioned in the handpiece body part, and connected to a suction port of the negative pressure formation space and a vacuum device of a control body part; and a cream movement guide member, which is mounted at the suction port in the negative pressure formation space so as to guide, toward the lower side thereof, treatment cream moving toward the suction port along the inner surface of the negative pressure formation space and to block the cream from flowing into the suction port, and thus the present invention blocks the treatment cream from being suctioned into an air flow path by negative pressure during a treatment so as to prevent a clogging accident of the air flow path, thereby ensuring operation stability, increasing the durability of the vacuum device, and reducing the costs and time required to repair the air flow path clogged by the treatment cream.

## Description

### Technical Field

The present disclosure relates to a suction handpiece and a skin care device including the suction handpiece. More particularly, the present disclosure relates to a suction handpiece configured to prevent an air flow path from being clogged by treatment cream during vacuum suction, and relates to a skin care device including the suction handpiece.

### Background Art

Generally, a skin care device using negative pressure provides a therapeutic effect by positioning a suction handpiece at a specific portion of a patient's skin and then providing negative pressure to the skin by using a pump, and use of such a skin care device is gradually increasing due to diversification of current skin treatments.

Therapeutic medical devices using negative pressure include a massage device using vacuum, a fat decomposition device that suctions a patient's skin by using vacuum and irradiates the suctioned portion with ultrasonic waves to decompose fat, a cupping device that suctions skin to acquire an oriental medical treatment effect.

In addition, a suction handpiece may include an RF (radio frequency) electrode and may provide a massage effect using negative pressure and a skin care effect by the RF electrode.

The RF electrode is used to treat skin by heating the skin and subcutaneous tissue, and is used for a purpose of restoring skin by increasing temperature of the skin after a cryolipolysis treatment for obesity treatment, and for skin treatment that causes collagen contraction or induces a wound healing response by heating the skin and the subcutaneous tissue.

More specifically, the RF (radio frequency) electrode is currently being variously used for skin remodeling/resurfacing, wrinkle removal, and treatment of sebaceous glands, hair follicle adipose tissue, and spider veins by applying an electrical signal to cause collagen in the lower dermis to undergo contraction or a wound healing response.

The suction handpiece is connected to a vacuum device through a suction pipe in order to form negative pressure therein, and alternately and repeatedly forms negative pressure and positive pressure in a negative pressure formation space in contact with the skin through the suction pipe.

In addition, the suction handpiece may perform a treatment after treatment cream is applied to the skin in order to enhance an effect during the treatment. In this case, there is a problem in that the treatment cream applied to the skin is suctioned, by negative pressure during the treatment, into an air flow path of the suction pipe through a suction port while the treatment cream is moving along an inner surface of the negative pressure formation space.

Particularly, in the suction handpiece provided with an RF (radio frequency) electrode, treatment cream is essentially applied in order to reduce pain and to minimize side effects when electrical stimulation is applied to the skin by the RF (radio frequency) electrode. Therefore, the treatment cream is frequently suctioned into the air flow path of the suction pipe through the suction port while the treatment cream is moving along the inner surface of the negative pressure formation space during the treatment.

When the treatment cream is suctioned into the air flow path of the suction pipe, negative pressure cannot be uniformly applied, thereby reducing a treatment effect. Furthermore, there is a problem in that the treatment cream remaining in the air flow path hardens and blocks the flow path of the suction pipe.

In addition, the treatment cream suctioned into the air flow path reduces durability of the vacuum device. Furthermore, when the treatment cream blocks the flow path of the suction pipe, an accident in which the treatment is stopped during the treatment occurs, thereby causing a problem in that time and costs are required to solve the accident.

In a conventional suction handpiece provided with an RF (radio frequency) electrode, a replaceable filter is mounted at the suction port in order to prevent treatment cream from being suctioned into the air flow path during the treatment. However, there are problems in that the filter is required to be periodically replaced, negative pressure and positive pressure are reduced by the filter, and as foreign substances are caught in the filter, the negative pressure and the positive pressure are reduced, thereby making the negative pressure and the positive pressure difficult to be constantly maintained at set pressures.

As a patent document in the related art of the present disclosure, there is Korean Patent Application Publication No. 10-2021-0147811 entitled "TREATMENT DEVICE USING VACUUM CUP" (published on December 7, 2021).

### Disclosure

### Technical Problem

An objective of the present disclosure is to provide a suction handpiece configured to prevent treatment cream from being suctioned into an air flow path by negative pressure during a treatment without using a filter, and is to provide a skin care device including the suction handpiece.

Another objective of the present disclosure is to provide a suction handpiece capable of efficiently generating negative pressure and positive pressure in a negative pressure formation space, and is to provide a skin care device including the suction handpiece.

### Technical Solution

In order to achieve the objectives described above, according to an aspect of the present disclosure, there is provided a suction handpiece including: a handpiece body part provided with a negative pressure formation space open toward a surface that comes into contact with skin; an air suction pipe member which is positioned in the handpiece body part and which connects a suction port of the negative pressure formation space and a vacuum device of a control body part to each other; and a cream movement guide member mounted on the suction port in the negative pressure formation space and configured to guide treatment cream which moves toward the suction port along an inner surface of the negative pressure formation space to flow downward, thereby blocking the treatment cream from being introduced into the suction port.

In the present disclosure, the cream movement guide member may be coupled to the suction port such that the cream movement guide member is capable of being separated from the suction port.

In the present disclosure, a coupling pipe part to which the cream movement guide member is coupled such that the cream movement guide member is capable of being separated from the coupling pipe part and which is formed on an upper portion of the negative pressure formation space may protrude from the suction port.

In the present disclosure, the cream movement guide member may include: a coupling protrusion part coupled to the suction port; and a cream support part formed so as to surround an outer circumference of the coupling protrusion part and configured such that the treatment cream moving downward along an outer surface of the coupling protrusion part is placed on an upper portion of the cream support part.

In the present disclosure, a coupling pipe part to which the cream movement guide member is coupled such that the cream movement guide member is capable of being separated from the coupling pipe part and which is formed on an upper portion of the negative pressure formation space may protrude from the suction port, and the cream movement guide member may be coupled to the coupling pipe part such that the coupling pipe part is fitted into an inner portion of the coupling protrusion part.

In the present disclosure, an end portion of the coupling protrusion part may be positioned in contact with an upper surface of the negative pressure formation space.

In the present disclosure, the cream support part may have an end portion of an upper surface thereof formed as a first inclined surface inclined downward so as to move the treatment cream downward.

In the present disclosure, the cream support part may have a lower surface thereof formed as a second inclined surface configured to collect air toward the suction port.

In the present disclosure, the cream support part may have an end portion of an upper surface thereof formed as a first inclined surface inclined downward so as to move the treatment cream downward, and may have a lower surface thereof formed as a second inclined surface configured to collect air toward the suction port.

In the present disclosure, a cream storage part configured to store the treatment cream flowing down along the coupling protrusion part may be positioned on an upper surface of the cream support part.

In the present disclosure, an inclined surface for guiding cream movement may be provided at an upper end portion of the coupling protrusion part, the inclined surface for guiding cream movement being configured to guide the treatment cream moving upward along the inner surface of the negative pressure formation space to the outer surface of the coupling protrusion part.

In the present disclosure, the cream movement guide member may be coupled to an upper portion of the negative pressure formation space such that the cream movement guide member is capable of being moved upward and downward, may be elastically supported by a spring member such that the cream movement guide member is moved upward and downward when negative pressure and positive pressure are alternately and repeatedly formed in the negative pressure formation space.

In the present disclosure, the cream movement guide member may be configured such that the coupling protrusion part penetrates an upper surface of the negative pressure formation space and is coupled to the upper surface of the negative pressure part so that the coupling protrusion part is capable of being moved upward and downward, and a spring support part elastically supporting the spring member may protrude from an inner surface of the coupling protrusion part. Furthermore, the spring member may be positioned between the spring support part and the upper surface of the negative pressure formation space, and may elastically support the cream movement guide member.

In the present disclosure, the spring support part may be positioned at a lower end portion on the inner surface of the coupling protrusion part, may be provided with a spring insertion groove part into which the spring member is inserted, and may have an upper end portion supported by the upper surface of the negative pressure formation space during upward movement of the cream movement guide member, thereby limiting an upward movement range of the cream movement guide member.

In the present disclosure, a lifting guide pipe part through which the coupling protrusion part penetrates and is coupled and which guides upward and downward movement of the coupling protrusion part may protrude from the upper surface of the negative pressure formation space, and a stopper protrusion part configured to be caught by an upper end portion of the lifting guide pipe part may protrude outward at an upper end portion of the coupling protrusion part.

In the present disclosure, an inflow blocking O-ring part configured to block the treatment cream positioned on the outer surface of the coupling protrusion part from being introduced into an inner side of the lifting guide pipe part may be positioned on an inner surface of the lifting guide pipe part.

In order to achieve the objectives described above, according to an aspect of the present disclosure, there is provided a skin care device including: a suction handpiece; and a control body part which is connected to the suction handpiece and which includes a vacuum device connected to a negative pressure formation space of the suction handpiece, wherein the suction handpiece is an aspect of the suction handpiece of the present disclosure.

### Advantageous Effects

In the present disclosure, the treatment cream is prevented from being suctioned into an air flow path by negative pressure during a treatment and a clogging accident of the air flow path is prevented, so that there are effects that operation stability is ensured, durability of the vacuum device is increased, and costs and time required to repair the air flow path clogged by the treatment cream are reduced.

In the present disclosure, the treatment cream is prevented from being suctioned into the air flow path by negative pressure during a procedure without using a filter, and negative pressure and positive pressure are constantly maintained at set pressures during the treatment, so that there are effect that a treatment effect is increased and costs and time required for equipment management are significantly reduced since a filter is not required to be replaced or managed.

### Description of Drawings

FIG. 1 is a schematic view illustrating a skin care device including a suction handpiece according to the present disclosure.
FIG. 2 is a cross-sectional view illustrating an embodiment of the suction handpiece according to the present disclosure.
FIG. 3 and FIG. 4 are cross-sectional views illustrating other embodiments of the suction handpiece according to the present disclosure.
FIG. 5 is a cross-sectional view illustrating still another embodiment of the suction handpiece according to the present disclosure.

### *DESCRIPTION OF REFERENCE NUMERALS OF IMPORTANT PARTS

10: Suction handpiece 20: Control body part
30: Handpiece cable body 100: Handpiece body part
100a: Negative pressure formation space 100b: Suction port
110: Cup part 111: Roof part
112: Cylindrical part 120: Coupling pipe part
130: Lifting guide pipe part 200: Air suction pipe member
300: Cream movement guide member 310: Coupling protrusion part
311: Spring support part 312: Stopper protrusion part
320: Cream support part 320a: First inclined surface
320b: Second inclined surface 321: Cream storage part
323: Inclined surface for guiding cream movement 400: RF electrode part
500: Spring member 600: Inflow blocking O-ring part

### Mode for Invention

Hereinafter, exemplary embodiments of the present disclosure will be described in detail with reference to the accompanying drawings. However, the present disclosure may be embodied in different forms and should not be construed as limited to the embodiments set forth herein. Rather, the embodiments introduced herein are provided so that disclosed contents will be thorough and complete, and will fully convey the scope of the present disclosure to those skilled in the art.

In the present specification, it will be understood that when an element is referred to as being "on" another element, it can be formed directly on the other element or intervening elements may be present. In addition, in the drawings, the shapes and the thicknesses of regions are exaggerated for clarity.

In addition, it will be also understood that although the terms first, second, third, and so on may be used herein to describe various elements, these elements should not be limited by these terms. These terms are only used to distinguish one element from another element. Thus, a first element in some embodiments may be termed a second element in other embodiments without departing from the teachings of the present disclosure. Embodiments explained and illustrated herein include their complementary counterparts. As used herein, the term "and/or" includes any and all combinations of one or more of the associated listed elements.

The singular expression also includes the plural meaning as long as it does not differently mean in the context. In addition, the terms "comprise", "have", and so on of the description are used to indicate that there are features, numbers, steps, elements, or combination thereof, and they should not exclude the possibilities of combination or addition of one or more features, numbers, operations, elements, or a combination thereof. Furthermore, it will be understood that when an element is referred to as being "connected" or "coupled" to another element, it may be directly connected or coupled to the other element or intervening elements may be present.

In addition, when detailed descriptions of related known functions or constitutions are considered to unnecessarily cloud the gist of the present disclosure in describing the present disclosure below, the detailed descriptions will not be included.

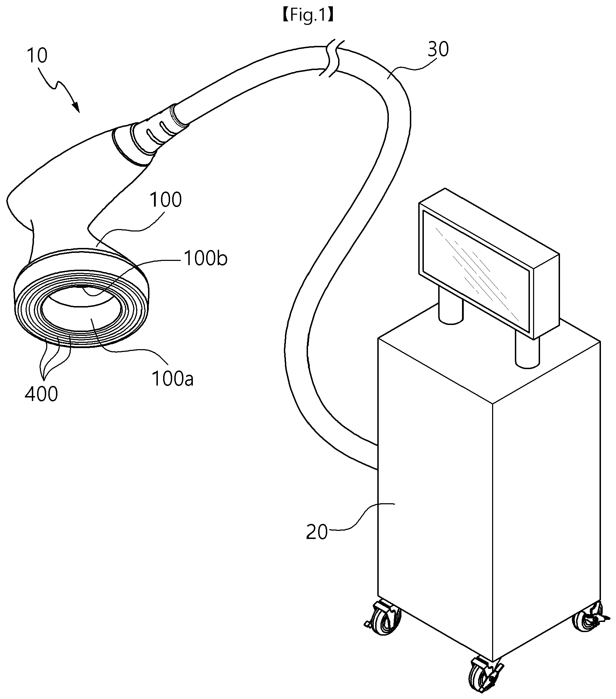

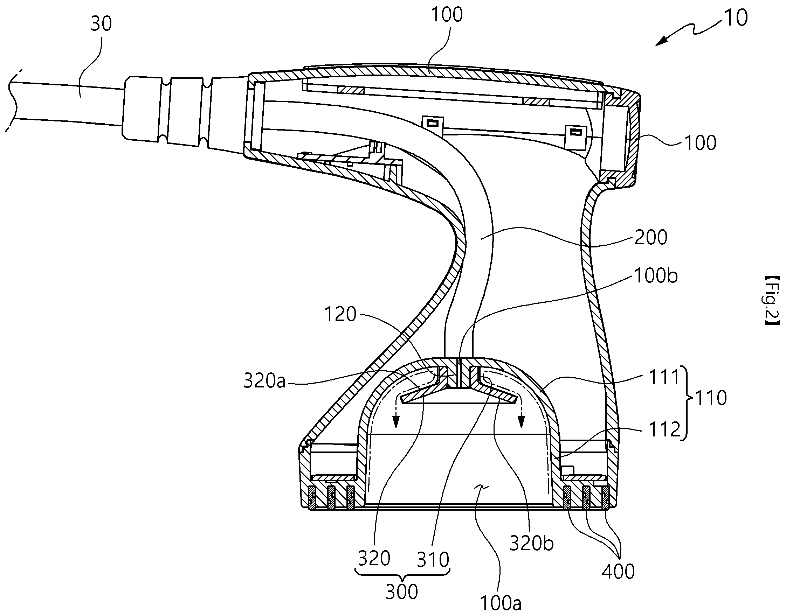

FIG. 1 is a schematic view illustrating a skin care device including a suction handpiece 10 according to the present disclosure, and FIG. 2 is a cross-sectional view illustrating an embodiment of the suction handpiece 10 according to the present disclosure.
an embodiment of the suction handpiece 10 according to the present disclosure and the skin care device including the suction handpiece 10 will be described in detail below with reference to FIG. 1 and FIG. 2.

The skin care device including the suction handpiece 10 according to the present disclosure includes the suction handpiece 10 according to the present disclosure, and includes a control body part 20 which is connected to the suction handpiece 10 and which includes a vacuum device connected to a negative pressure formation space 100a of the suction handpiece 10.

The suction handpiece 10 according to the present disclosure may include the negative pressure formation space 100a configured to be seated on the skin and open toward a surface that comes into contact with the skin, and may include an RF electrode part 400 configured to come into contact with the skin and to apply an RF electrical signal to the skin.

The control body part 20 is connected to the suction handpiece 10 by a handpiece cable body 30 including a control cable, an air suction and exhaust line, and so on, and includes the vacuum device capable of repeatedly forming negative pressure and positive pressure in the negative pressure formation space 100a of the suction handpiece 10.

The control cable is a cable for controlling operation of the RF electrode part 400, and the air suction and exhaust line includes a pipe member which is connected to the vacuum device of the control body part 20 and which is configured to suction air in the negative pressure formation space 100a or to supply air into the negative pressure formation space 100a.

The control body part 20 and the handpiece cable body 30 may be variously modified and implemented as examples known in a known negative-pressure treatment device that controls operation of the suction handpiece 10 including an RF electrode, so that a more detailed description thereof will be omitted.

Meanwhile, an embodiment of the suction handpiece 10 according to the present disclosure includes a handpiece body part 100 provided with the negative pressure formation space 100a open toward a surface that comes into contact with the skin.

An inner portion of the handpiece body part 100 is provided with the negative pressure formation space 100a open toward a lower side, i.e., toward a contact surface that comes into contact with the lower side.

The handpiece body part 100 includes a cup part 110 in which the negative pressure formation space 100a is formed. As an example, the cup part 110 has a shape which includes a roof part 111 having a hemispherical dome shape and which includes a cylindrical part 112 positioned at a lower side of the roof part 111, and has a structure in which a lower end side of the cylindrical part 112 is connected to the contact surface that is a lower surface of the handpiece body part 100 and comes into contact with the skin.

The negative pressure formation space 100a may also be variously modified and implemented in known shapes in the suction handpiece 10, so that a more detailed description thereof will be omitted.

In addition, a suction port 100b for forming negative pressure by suctioning air therein or forming positive pressure by supplying air thereinto is positioned in the negative pressure formation space 100a.

As an example, the suction port 100b is positioned at a center of the negative pressure formation space 100a, i.e., at a central portion of the roof part 111. As the suction port 100b is positioned at the center of the roof part 111, air in the negative pressure formation space 100a may be uniformly suctioned and air may be uniformly supplied into the negative pressure formation space 100a.

The suction port 100b is connected to the vacuum device of the control body part 20 through an air suction pipe member 200 positioned in the handpiece body part 100.

The air suction pipe member 200 may be integrally configured as one line with the air suction and exhaust line of the handpiece cable body 30, or may have a structure capable of being separated from the air suction and exhaust line of the handpiece cable body 30.

In addition, as an example, a plurality of RF electrode parts 400 is positioned on the contact surface of the handpiece body part 100, and each of the RF electrode parts 400 has a ring shape surrounding an inlet of the negative pressure formation space 100a.

In addition, the RF electrode part 400 may include a plurality of RF electrodes having ring shapes and positioned to be spaced apart from each other on the contact surface of the handpiece body part 100.

As an example, the plurality of RF electrode parts 400 is ring-shaped electrodes having different diameters, and is positioned to be spaced apart from each other on the contact surface of the handpiece body part 100 while having the same width as each other or different widths from each other.

Although not illustrated, the RF electrode part 400 may be positioned on an inner surface of the negative pressure formation space 100a so as to be positioned to come into contact with a skin portion that protrudes into the negative pressure formation space by negative pressure.

In summary, the RF electrode part 400 is positioned on the handpiece body part 100 so as to come into contact with the skin, and applies an RF electrical signal to the contacted skin.

The RF electrode part 400 is a known RF electrode variously used for skin remodeling/resurfacing, wrinkle removal, and treatment of sebaceous glands, hair follicle adipose tissue, and spider veins by applying an electrical signal to cause collagen in the lower dermis to undergo contraction or a wound healing response, so that a more detailed description thereof will be omitted.

A control wire part (not illustrated) connecting the RF electrode part 400 and the control body part 20 to each other and controlling operation of the RF electrode part 400 is provided in the handpiece body part 100.

The control wire part may be integrally configured as one line with the control cable of the handpiece cable body 30, or may have a structure capable of being separated from the control cable of the handpiece cable body 30.

A cream movement guide member 300 is mounted at the suction port 100b of the negative pressure formation space 100a, the cream movement guide member 300 being configured to guide treatment cream moving toward the suction port 100b along the inner surface of the negative pressure formation space 100a to be moved downward so as to block the treatment cream from flowing into the suction port 100b.

During a treatment, the treatment cream applied to the skin moves toward the suction port 100b formed on an upper surface of the negative pressure formation space 100a along the inner surface of the negative pressure formation space 100a, and the cream movement guide member 300 guides the treatment cream to be moved downward, i.e., toward the skin.

The cream movement guide member 300 is provided at the suction port 100b positioned at an upper side of the negative pressure formation space 100a, and guides the treatment cream which is moved toward the upper side of the negative pressure formation space 100a along the negative pressure formation space 100a to flow downward along an outer surface of the cream movement guide member 300, thereby preventing the treatment cream from being suctioned into the suction port 100b.

The treatment cream may be negative-pressure treatment cream used during a negative-pressure treatment, or may be RF treatment cream used during an RF treatment.

Generally, during an RF treatment, RF treatment cream is essentially applied in order to reduce pain and to minimize side effects, and the RF treatment cream may move toward the suction port 100b along the inner surface of the negative pressure formation space 100a and may be introduced into the suction port 100b during the treatment.

As an example, in the suction handpiece 10 that performs an RF treatment and a negative-pressure treatment together, the cream movement guide member 300 blocks RF treatment cream moving toward the suction port 100b along the inner surface of the negative pressure formation space 100a during the treatment from being introduced into the suction port 100b.

The cream movement guide member 300 includes a coupling protrusion part 310 coupled to the suction port 100b, and includes a cream support part 320 which is formed so as to surround an outer circumference of the coupling protrusion part 310 and which has an upper surface where the treatment cream moved downward along an outer surface of the coupling protrusion part 310 is placed.

As an example, the coupling protrusion part 310 has a tubular shape having a hollow in a length direction, and is inserted into an inner portion of the suction port 100b or is fitted to an outer portion of the suction port 100b to be coupled thereto, thereby having a structure that is easily separable.

More specifically, a coupling pipe part 120 to which the cream movement guide member 300 is separably coupled is formed on the upper portion of the negative pressure formation space 100a such that the coupling pipe part 120 protrudes from the suction port 100b. As an example, the cream movement guide member 300 is coupled to the coupling pipe part 120 such that the coupling pipe part 120 is inserted into and coupled to an inner portion of the coupling protrusion part 310.

In addition, an end portion of the coupling protrusion part 310 is positioned in contact with the upper surface of the negative pressure formation space 100a, i.e., the upper surface of the cup part 110, thereby preventing the treatment cream from being introduced and caught between the end portion of the coupling protrusion part 310 and the negative pressure formation space 100a.

The coupling pipe part 120 is inserted into and coupled to the hollow portion of the coupling protrusion part 310 and has a structure in which a diameter of the suction port 100b is not reduced. Since the end portion of the coupling protrusion part 310 is positioned in contact with the upper surface of the negative pressure formation space 100a, the treatment cream moved up to the upper surface of the negative pressure formation space 100a smoothly moves downward without being caught between the end portion of the coupling protrusion part 310 and the negative pressure formation space 100a when the treatment cream moves downward along an outer circumferential surface of the coupling protrusion part 310.

That is, the cream movement guide member 300 includes the coupling protrusion part 310 which is coupled to the suction port 100b and which protrudes downward from the suction port 100b, thereby guiding the treatment cream moved up to the upper surface of the negative pressure formation space 100a to be moved downward along the outer surface of the cream movement guide member 300.

In addition, the cream support part 320 on which the treatment cream moving downward is positioned on the upper surface of the cream support part 320 is provided around the outer circumference of the coupling protrusion part 310.

The cream support part 320 extends an area at an end side of the coupling protrusion part 310 that guides the treatment cream to be moved downward, thereby preventing the treatment cream from being introduced into the hollow portion of the coupling protrusion part 310 when air is suctioned through the suction port 100b at the end portion of the coupling protrusion part 310.

The cream support part 320 is formed so as to have an area that prevents the treatment cream positioned around the outer circumference of the cream support part 320 by suction pressure through the suction port 100b from being reintroduced toward the suction port 100b.

As an example, the cream support part 320 has a diameter at least twice a diameter of the coupling protrusion part 310 or has a diameter at least twice a diameter of the suction port 100b, thereby preventing the treatment cream from being reintroduced toward the suction port 100b.

As an example, the cream support part 320 is formed in a disk shape surrounding the outer circumference of the coupling protrusion part 310.

In addition, an upper surface of the cream support part 320 is formed as an inclined surface inclined such that an end portion of the upper surface of the cream support part 320 is directed downward, thereby guiding the treatment cream placed on the upper side of the cream support part 320 to be moved in a direction toward the end side of the cream support part 320.

In addition, a lower surface of the cream support part 320 is formed as an inclined surface having a shape that spreads from the suction port 100b side toward the end side of the cream support part 320.

That is, as an example, the cream support part 320 has a funnel shape in which an end portion of the upper surface thereof is formed as a first inclined surface 320a inclined downward so as to move the treatment cream downward, and the lower surface is formed as a second inclined surface 320b that collects air toward the suction port 100b.

The cream support part 320 guides air in the negative pressure formation space 100a toward the suction port 100b by the second inclined surface 320b at the lower side of the cream support part 320 during formation of negative pressure so that the air can be smoothly suctioned, and supplies air supplied into the negative pressure formation space 100a during formation of positive pressure so as to spread the air in a wide direction. Accordingly, when negative pressure and positive pressure are repeatedly formed, preset negative pressure and positive pressure may be reached as quickly as possible, and cycles of positive pressure and negative pressure may be shortened.

In addition, the cream support part 320 guides the treatment cream that has moved along the coupling protrusion part 310 to the upper surface of the cream support part 320 to be moved downward along the inclined first inclined surface 320a, so that the treatment cream is dropped from the end portion of the first inclined surface 320a.

The cream support part 320 may smoothly move the treatment cream downward through the first inclined surface 320a at the upper portion of the cream support part 320, may drop the treatment cream back onto the skin, and may return the treatment cream to an original position, thereby minimizing replenishment of the treatment cream during the treatment and minimizing an amount of treatment cream remaining in the negative pressure formation space 100a after the treatment.

In summary, as negative pressure and positive pressure are alternately and repeatedly generated in the negative pressure formation space 100a during the treatment, the treatment cream applied to the skin moves toward the suction port 100b positioned on the upper surface of the negative pressure formation space 100a along the inner surface of the negative pressure formation space.

After the treatment cream is moved to the suction port 100b side, the treatment cream is guided downward along the outer surface of the coupling protrusion part 310 that protrudes from the suction port 100b and the treatment cream is moved onto the cream support part 320, and the treatment cream may be moved downward along the first inclined surface 320a from the upper surface of the cream support part 320 and is dropped back onto the skin.

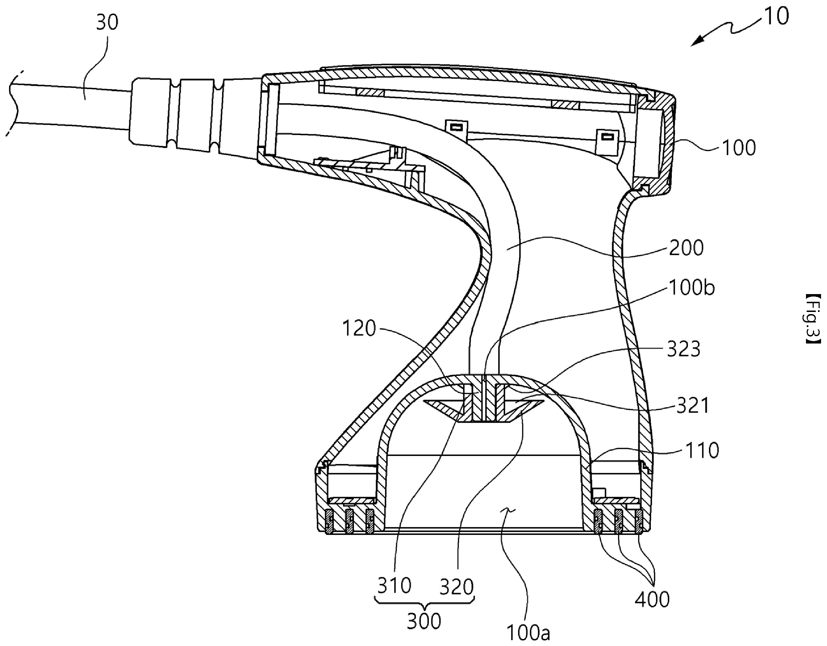

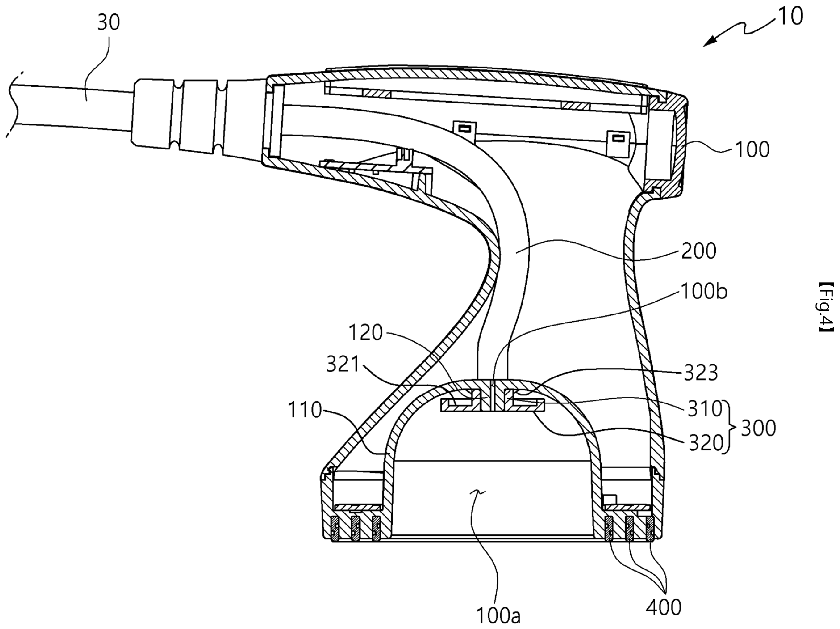

FIG. 3 and FIG. 4 are cross-sectional views illustrating other embodiments of the suction handpiece 10 according to the present disclosure. Referring to FIG. 3 and FIG. 4, a cream storage part 321 that stores the treatment cream flowing down along the coupling protrusion part 310 is positioned on the upper surface of the cream support part 320, and the treatment cream that has been used and has moved upward along the inner surface of the negative pressure formation space 100a is stored and kept in the cream storage part 321, thereby blocking the treatment cream from being introduced into the suction port 100b.

In a in which a treatment for removing body wastes in the skin, the body wastes in the skin are mixed with and present in the treatment cream. In a case of the treatment cream mixed with the body wastes, the treatment cream may be stored and kept in the cream storage part 321 formed on the upper surface of the cream support part 320 without being dropped back onto the skin and reused. After the treatment, the cream movement guide member 300 may be separated from the suction port 100b, may be washed, and then may be coupled to the suction port 100b again for use.

As illustrated in FIG. 3, the cream movement guide member 300 may have a hopper shape having a diameter that increases toward an upper side thereof, and may have a structure in which the cream storage part 321 is formed in the hopper.

In addition, as illustrated in FIG. 4, the cream movement guide member 300 may have a structure in which the cream storage part 321 recessed around the coupling protrusion part 310 is formed on the upper surface of the cream storage part 321.

The cream movement guide member 300 may have various forms of the cream storage part 321 on the upper surface thereof, the cream storage part 321 being capable of storing the treatment cream that has moved down along the outer surface of the coupling protrusion part 310.

In addition, the cream movement guide member 300 may be manufactured in various shapes suitable for a type of treatment, may be separably coupled to the suction port 100b, and may be used by coupling a shape suitable for the treatment to the suction port 100b.

As negative pressure and positive pressure are alternately and repeatedly generated in the negative pressure formation space 100a during the treatment, the treatment cream applied to the skin moves toward the suction port 100b positioned on the upper surface of the negative pressure formation space 100a along the inner surface of the negative pressure formation space.

After the treatment cream is moved to the suction port 100b side, the treatment cream is guided downward along the outer surface of the coupling protrusion part 310 that protrudes from the suction port 100b and the treatment cream is moved onto the cream support part 320, and the treatment cream may be moved downward along the first inclined surface 320a from the upper surface of the cream support part 320 and is dropped back onto the skin.

In addition, an inclined surface 323 for guiding cream movement, which guides the treatment cream that has moved upward along the inner surface of the negative pressure formation space 100a to the outer surface of the coupling protrusion part 310, is provided at an upper end portion of the coupling protrusion part 310.

The inclined surface 323 for guiding cream movement is formed in a cone shape having a diameter that gradually increases from the upper end portion of the coupling protrusion part 310, surrounds the outer circumference of the suction port 100b, and is in close contact with the upper surface of the negative pressure formation space 100a.

The inclined surface 323 for guiding cream movement guides the treatment cream that has moved upward toward the suction port 100b along the inner surface of the negative pressure formation space 100a to the outer surface of the coupling protrusion part 310, thereby allowing the treatment cream to move smoothly and preventing the treatment cream from being caught and accumulated at a specific portion during movement.

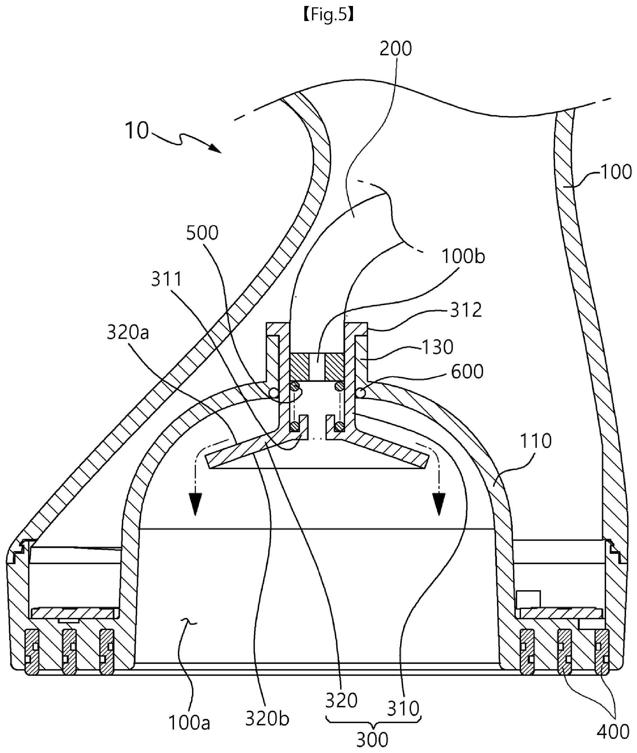

In addition, FIG. 5 is a cross-sectional view illustrating still another embodiment of the suction handpiece 10 according to the present disclosure. Referring to FIG. 5, the cream movement guide member 300 is coupled to the upper side of the negative pressure formation space 100a so as to be movable upward and downward, is elastically supported by a spring member 500, so that the cream guide movement member 300 is configured to be moved upward and downward when negative pressure and positive pressure are alternately and repeatedly formed in the negative pressure formation space 100a.

More specifically, the cream movement guide member 300 is coupled such that the coupling protrusion part 310 penetrates the upper surface of the negative pressure formation space 100a so that the cream movement guide member 300 is capable of being moved upward and downward. Furthermore, a spring support part 311 that elastically supports the spring member 500 protrudes from an inner surface of the coupling protrusion part 310, and the spring member 500 is positioned between the spring support part 311 and the upper surface of the negative pressure formation space 100a, so that the spring member 500 elastically supports the cream movement guide member 300.

The spring support part 311 is positioned at a lower end side on the inner surface of the coupling protrusion part 310, and is provided with a spring insertion groove part into which the spring member 500 is inserted. In addition, during upward movement of the cream movement guide member 300, an upper end side of the spring support part 311 is supported by the upper surface of the negative pressure formation space 100a, and serves as a stopper for limiting an upward movement range of the cream movement guide member 300.

In addition, a lifting guide pipe part 130 through which the coupling protrusion part 310 penetrates and is coupled and which guides upward and downward movement of the coupling protrusion part 310 is protrudes from the upper surface of the negative pressure formation space 100a, and a stopper protrusion part 312 caught by an upper end portion of the lifting guide pipe part 130 protrudes outward at an upper end portion of the coupling protrusion part 310.

During downward movement of the cream movement guide member 300, the stopper protrusion part 312 serves as a stopper for limiting a downward movement range as an upper end portion of the stopper protrusion part 312 is caught by the upper end portion of the lifting guide pipe part 130.

The cream movement guide member 300 is configured to be moved upward and downward as negative pressure and positive pressure are alternately and repeatedly generated, and is moved upward and downward between a position at which the spring support part 311 is caught by the upper surface of the negative pressure formation space 100a during upward movement and a position at which the stopper protrusion part 312 is caught by the lifting guide pipe part 130 during downward movement.

In addition, an inflow blocking O-ring part 600 configured to block the treatment cream positioned on the outer surface of the coupling protrusion part 310 from being introduced into an inner side of the lifting guide pipe part 130 is positioned on an inner surface of the lifting guide pipe part 130.

The inflow blocking O-ring part 600 is positioned at a lower end portion on the inner surface of the lifting guide pipe part 130, and is configured to block the treatment cream from being introduced into the inner side of the lifting guide pipe part 130.

When negative pressure is generated in the negative pressure formation space 100a, the cream movement guide member 300 is moved upward by air being suctioned, i.e., by the negative pressure. Furthermore, when positive pressure is generated, the cream movement guide member 300 is moved downward by air discharged through the suction port 100b, i.e., by the positive pressure.

During upward movement of the cream movement guide member 300, the treatment cream moving along the outer surface of the coupling protrusion part 310 is pushed downward from the upper surface of the negative pressure formation space 100a and is moved more quickly to the cream support part 320.

After the treatment cream is moved to the outer surface of the coupling protrusion part 310, the treatment cream is moved downward more quickly and smoothly during upward and downward movement of the cream movement guide member 300, thereby preventing the treatment cream from becoming stagnant at a specific portion and hardening and accumulating.

In addition, vibration is generated in the cream support part 320 by the upward and downward movement of the cream movement guide member 300. Accordingly, the treatment cream moving on the first inclined surface 320a that is the upper surface of the cream support part 320 is moved smoothly and quickly downward along the first inclined surface 320a by the vibration, and may drop back onto the skin.

The cream movement guide member 300 is moved upward and downward when negative pressure and positive pressure are generated, and quickly moves the treatment cream moving upward toward the suction port 100b along the inner surface of the negative pressure formation space 100a downward again and resupplies the treatment cream onto the skin. Accordingly, circulation of the treatment cream in the negative pressure formation space 100a is quickly performed during the treatment, and the treatment may be performed without requiring replenishment of the treatment cream due to loss of the treatment cream.

The present disclosure prevents the treatment cream from being suctioned into an air flow path by negative pressure during a treatment and prevents a clogging accident of the air flow path, thereby ensuring operation stability, increasing durability of the vacuum device, and reducing costs and time required to repair the air flow path clogged by the treatment cream.

The present disclosure prevents the treatment cream from being suctioned into the air flow path by negative pressure during a treatment without using a filter, and allows negative pressure and positive pressure to be constantly maintained at set pressures during the treatment, thereby increasing a treatment effect and significantly reducing costs and time required for equipment management since a filter is not required to be replaced or managed.

While the present disclosure has been described in connection with the embodiments, it is not to be limited thereto but will be defined by the appended claims. In addition, it is to be understood that those skilled in the art can substitute, change or modify the embodiments in various forms without departing from the scope and spirit of the present disclosure.

## Claims

1. A suction handpiece comprising:
a handpiece body part provided with a negative pressure formation space open toward a surface that comes into contact with skin;
an air suction pipe member which is positioned in the handpiece body part and which connects a suction port of the negative pressure formation space and a vacuum device of a control body part to each other; and
a cream movement guide member mounted on the suction port in the negative pressure formation space and configured to guide treatment cream which moves toward the suction port along an inner surface of the negative pressure formation space to flow downward, thereby blocking the treatment cream from being introduced into the suction port.

2. The suction handpiece of claim 1, wherein the cream movement guide member is coupled to the suction port such that the cream movement guide member is capable of being separated from the suction port.

3. The suction handpiece of claim 1, wherein a coupling pipe part to which the cream movement guide member is coupled such that the cream movement guide member is capable of being separated from the coupling pipe part and which is formed on an upper portion of the negative pressure formation space protrudes from the suction port.

4. The suction handpiece of claim 1, wherein the cream movement guide member comprises:
a coupling protrusion part coupled to the suction port; and
a cream support part formed so as to surround an outer circumference of the coupling protrusion part and configured such that the treatment cream moving downward along an outer surface of the coupling protrusion part is placed on an upper portion of the cream support part.

5. The suction handpiece of claim 4, wherein a coupling pipe part to which the cream movement guide member is coupled such that the cream movement guide member is capable of being separated from the coupling pipe part and which is formed on an upper portion of the negative pressure formation space protrudes from the suction port, and the cream movement guide member is coupled to the coupling pipe part such that the coupling pipe part is fitted into an inner portion of the coupling protrusion part.

6. The suction handpiece of claim 5, wherein an end portion of the coupling protrusion part is positioned in contact with an upper surface of the negative pressure formation space.

7. The suction handpiece of claim 4, wherein the cream support part has an end portion of an upper surface thereof formed as a first inclined surface inclined downward so as to move the treatment cream downward.

8. The suction handpiece of claim 4, wherein the cream support part has a lower surface thereof formed as a second inclined surface configured to collect air toward the suction port.

9. The suction handpiece of claim 4, wherein the cream support part has an end portion of an upper surface thereof formed as a first inclined surface inclined downward so as to move the treatment cream downward, and has a lower surface thereof formed as a second inclined surface configured to collect air toward the suction port.

10. The suction handpiece of claim 4, wherein a cream storage part configured to store the treatment cream flowing down along the coupling protrusion part is positioned on an upper surface of the cream support part.

11. The suction handpiece of claim 6, wherein an inclined surface for guiding cream movement is provided at an upper end portion of the coupling protrusion part, the inclined surface for guiding cream movement being configured to guide the treatment cream moving upward along the inner surface of the negative pressure formation space to the outer surface of the coupling protrusion part.

12. The suction handpiece of claim 4, wherein the cream movement guide member is coupled to an upper portion of the negative pressure formation space such that the cream movement guide member is capable of being moved upward and downward, is elastically supported by a spring member such that the cream movement guide member is moved upward and downward when negative pressure and positive pressure are alternately and repeatedly formed in the negative pressure formation space.

13. The suction handpiece of claim 12, wherein the cream movement guide member is configured such that the coupling protrusion part penetrates an upper surface of the negative pressure formation space and is coupled to the upper surface of the negative pressure part so that the coupling protrusion part is capable of being moved upward and downward, and a spring support part elastically supporting the spring member protrudes from an inner surface of the coupling protrusion part, and
wherein the spring member is positioned between the spring support part and the upper surface of the negative pressure formation space, and elastically supports the cream movement guide member.

14. The suction handpiece of claim 13, wherein the spring support part is positioned at a lower end portion on the inner surface of the coupling protrusion part, is provided with a spring insertion groove part into which the spring member is inserted, and has an upper end portion supported by the upper surface of the negative pressure formation space during upward movement of the cream movement guide member, thereby limiting an upward movement range of the cream movement guide member.

15. The suction handpiece of claim 13, wherein a lifting guide pipe part through which the coupling protrusion part penetrates and is coupled and which guides upward and downward movement of the coupling protrusion part protrudes from the upper surface of the negative pressure formation space, and
wherein a stopper protrusion part configured to be caught by an upper end portion of the lifting guide pipe part protrudes outward at an upper end portion of the coupling protrusion part.

16. The suction handpiece of claim 15, wherein an inflow blocking O-ring part configured to block the treatment cream positioned on the outer surface of the coupling protrusion part from being introduced into an inner side of the lifting guide pipe part is positioned on an inner surface of the lifting guide pipe part.

17. A skin care device comprising:
a suction handpiece; and
a control body part which is connected to the suction handpiece and which includes a vacuum device connected to a negative pressure formation space of the suction handpiece,
wherein the suction handpiece is the suction handpiece of any one of claim 1 to claim 16.
